# EUROPEAN PATENT APPLICATION

(11) **EP 4 273 218 A1**
(43) Date of publication of application: **08.11.2023**
(21) Application number: 21914282.5
(22) Date of filing: 27.12.2021
(51) Int. Cl.: C12M 1/42

(54) **FLOW TYPE ELECTROTRANSFECTION DEVICE**

(30) Priority: 29.12.2020 CN 202011587236
(71) Applicant: Suzhou Etta Biotech Co., Ltd., Suzhou, Jiangsu 215126 (CN)
(72) Inventor: DAI, Edward, Suzhou, Jiangsu 215126 (CN); ZHU, Shiying, Suzhou, Jiangsu 215126 (CN)
(74) Representative: Murgitroyd & Company
(86) International application number: PCT/CN2021/141722
(87) International publication number: WO 2022/143546

(57) **Abstract**

A flow electrotransfection device with a chamber for fluid to flow though, a liquid inlet channel for communicating with the chamber and a liquid outlet channel for communicating with the chamber, includes an electrode module for applying an electric field to fluid in the chamber, and further includes a fluid guiding structure for making flow velocities of fluid flowing through the central zone and the peripheral zone of the chamber tend to be consistent. The present invention solves the technical problem of relatively uneven the flow velocities of fluid at different zones in the flow electrotransfection chamber, improves the accuracy of the number of electric shock when the fluid flows though the chamber, thus improving the electrotransfection efficiency, and reduces the cell damage and even death from excessive times of electric shock, thereby improving cell viability.

## Description

The present invention claims the priority of the Chinese patent application CN202011587236.1 filed on December 29, 2020.

### Technical field

The present invention relates to a flow electrotransfection device, more specifically, to a device for regulating flow velocity of fluid in a flow electrotransfection device, and to a method for regulating flow velocities of fluid at different zones in the electrotransfection chamber by using a fluid guiding structure, to achieve an overall even flow velocity.

### Background

A cell membrane is a phospholipid bilayer that forms the outer layer of a cell, which is a permeable barrier for selective exchange of substances between a cell and its surrounding. The cell membrane makes the cell an independent living unit, with a relatively stable internal environment. Some substances in the surrounding environment can pass through the cell membrane, while others cannot. A cell can take in nutrients from the surrounding environment and excrete metabolites through the cell membrane, so that transport of substances reaches a balanced state. Therefore, the basic function of the cell membrane is to maintain the relative stability of the intracellular microenvironment and selectively exchange substances with the external environment.

Studies have suggested that if an electric field of a certain intensity is applied to a cell for a period of time, some micropores can be induced on the cell membrane to enhance permeability of the cell. The so-called cell electroporation refers to the biophysical process in which the cell forms transient micropores on the lipid bilayer/cell membrane, under the impact of an external pulsed electric field. When a cell membrane is electroporated, its permeability and membrane conductance will increase instantaneously, which allows hydrophilic molecules, nucleic acids, proteins, virus particles, drug particles and other substances that normally cannot pass through the cell membrane to enter the cell. After the electric field is removed after a short period of time, the cell membrane can self-recover and become a selective permeable barrier again. This process of introducing exogenous substances into cells by utilizing characteristic of electroporation of cell membrane is called cell electrotransfection.

Although mechanism of electrotransfection is not fully understood, it is well known herein that cell electrotransfection involves disruption of the lipid bilayer of the cell membrane, resulting in formation of temporary micropores on the membrane that allow entry of exogenous molecules into the cell.

According to previous research, to realize high-throughput cell electrotransfection, cell suspension is arranged to flow through the chamber, where planar electrodes or a needle electrode array is used to apply an electric field to cell suspension in the chamber, so that cells thereof receive electric shock. Typically, such a device is referred to as a flow electrotransfection device.

For the devices disclosed in current technologies, the distance between planar electrodes is usually around 0.2-0.8 cm, i.e. height of the electrotransfection chamber is not large, but length and width (0.5-1 cm) of electrodes tend to be relatively larger, i.e. length and width of the electrotransfection chamber are also relatively large. Length of the electrotransfection chamber containing a needle electrode array is also large. The characteristic of fluid causes uneven flow velocities of fluid at different zones when fluid flows through an electrotransfection chamber, especially through longer chambers.

During the operation of a flow electrotransfection device, cell suspension enters from the liquid inlet, receives electric shocks at a certain time interval from the electrodes in the chamber while flows through the electrotransfection chamber, and flows out from the liquid outlet.

The characteristic of fluid is that the flow velocity of fluid near central zone of an electrotransfection chamber is faster, and the flow velocity of fluid near the chamber wall is slower.

Uneven flow velocity of fluid in an electrotransfection chamber will cause cells to receive different number of electric shock, which affects the electrotransfection efficiency. If local fluid retention occurs in the electrotransfection chamber, cells in the retention zone will receive repeated electric shock, resulting in death of cells in the retention zone, thereby reducing the overall cell viability.

Meanwhile, the fluid with a faster flow velocity in the electrotransfection chamber may receive relatively fewer times of electric shock, and cells thereof have lower transfection efficiency, thus reducing the overall cell transfection efficiency. Therefore, in the actual use of a flow electrotransfection device, users hope to adjust flow velocities of fluid at different zones in the electrotransfection chamber to keep velocities in different zones of the electroporation chamber as even as possible, so as to solve the above-mentioned technical problems of low electrotransfection efficiency and cell viability caused by uneven flow velocity of fluid. There are no current technologies for improving the unevenness of flow velocity of fluid in flow electrotransfection devices.

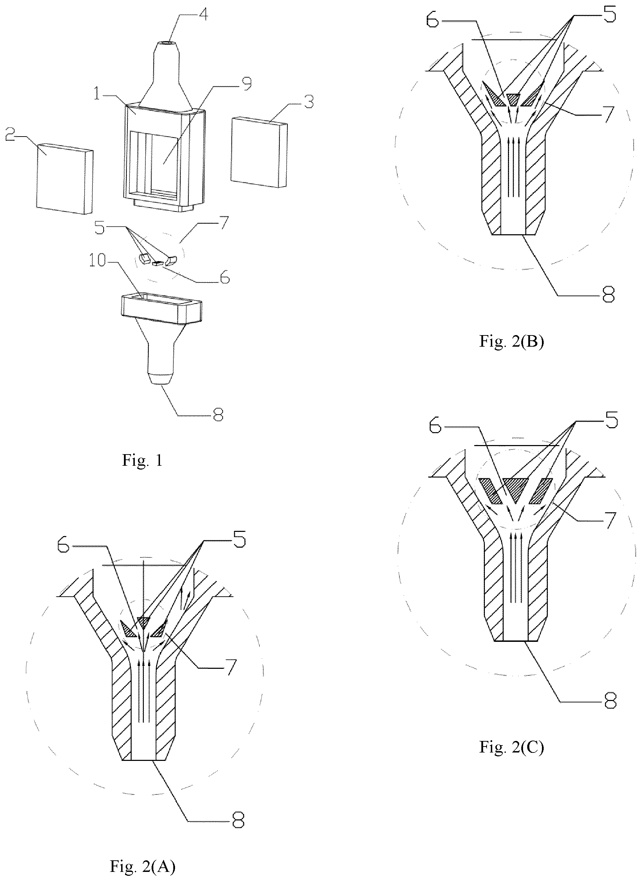

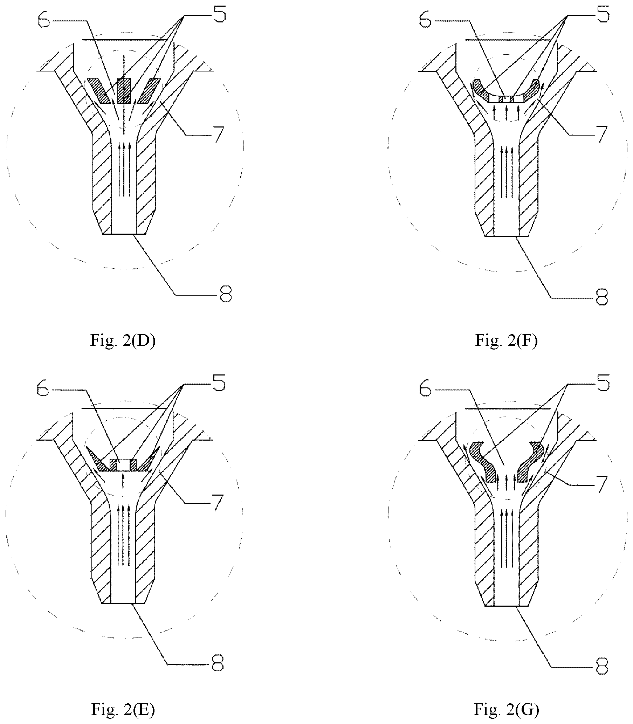

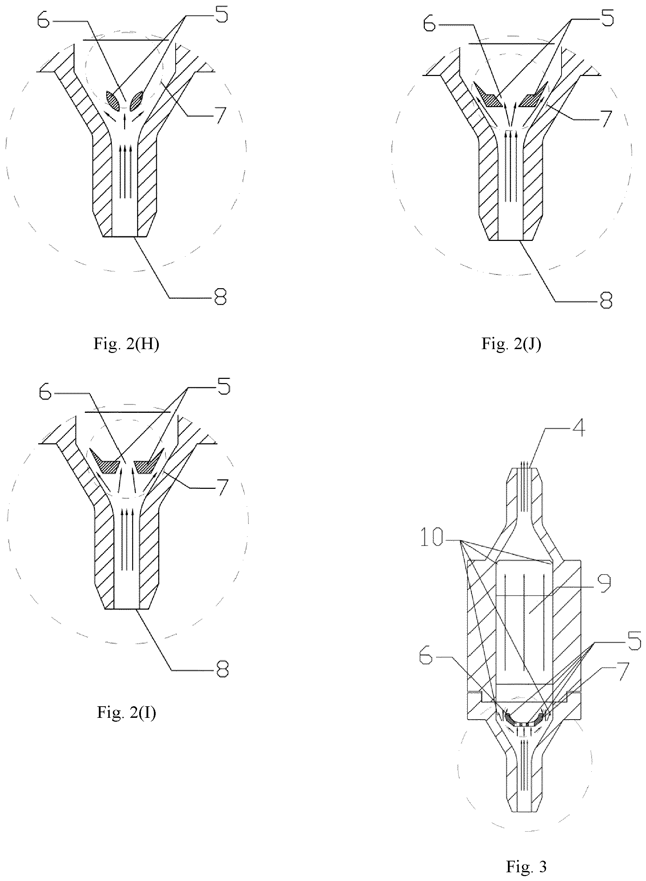

The present invention is aiming at solving the technical problem that the flow velocity of fluid in the chamber of a flow electrotransfection device is uneven, that is, the flow velocity of fluid in the central zone of the chamber is relatively faster, while the flow velocity of fluid at a zone near the chamber wall is relatively slower that results in a retention zone (Fig. 2).

### Summary of the invention

In order to overcome the above-mentioned deficiencies in the prior art, the present invention provides a flow electrotransfection device, in which the flow velocities of fluid at different zones in the electrotransfection chamber are relatively even, therefore cells passing through the different zones of the electrotransfection chamber receive approximately the same number of electric shock, hence greatly improving cell viability and electrotransfection efficiency.

To reach above-mentioned objectives, the present invention adopts following technical solutions:
A flow electrotransfection device with a chamber for fluid to flow though, a liquid inlet channel connected to the chamber and a liquid outlet channel connected to the chamber, includes an electrode module for applying an electric field to fluid in the chamber, and further includes a fluid guiding structure for evening out flow velocities of fluid flowing through different zones in the chamber.

In an embodiment, the fluid guiding structure, located in the liquid inlet channel, comprises a plurality of side fluid diverting blocks, and a central fluid diverting block located between the side fluid diverting blocks. Each of the side fluid diverting blocks and the central fluid diverting block includes an upstream end that is closer to the liquid inlet channel and a downstream end that is farther away from the liquid inlet channel. A first fissure for fluid to flow though is formed between the upstream end of the central fluid diverting block and the upstream end of the side fluid diverting blocks, and a second fissure for fluid to flow though is formed between the upstream end of the side fluid diverting block and the wall of the liquid inlet channel.

In a preferred embodiment, a third fissure, which is roughly equal in width to each of the second fissures, is formed between the upstream ends of the two side fluid diverting blocks.

In a preferred embodiment, the outer contours of the cross-sections of the side fluid diverting block and the central fluid diverting block consist of arcs.

In a preferred embodiment, the width of the cross-section of the side fluid diverting block gradually increases and then decreases from its upstream end to its downstream end, and the width of the cross-section of the central fluid diverting block gradually increases and then decreases from its upstream end to its downstream end.

In a preferred embodiment, the width of the cross-section of the central fluid diverting block changes specifically according to the following rule that the rate of change from the upstream end to the middle part is larger than that from the middle part to the downstream end. That is, the change in width from the middle part to the downstream end (i.e. its rear half) of the central fluid diverting block is more gentle than that from the upstream end to the middle part (i.e. its front half) of the central fluid diverting block.

In a preferred embodiment, the distance between the upstream ends of the side fluid diverting blocks is smaller than that between the downstream ends of the side fluid diverting blocks.

In a preferred embodiment, the geometric center of each of the side fluid diverting blocks is equidistant from the geometric center of the central fluid diverting block.

In a preferred embodiment, the liquid inlet channel and the liquid outlet channel are located at the front and rear sides of the chamber respectively, and the upstream end of the central fluid diverting block is located behind the upstream end of the side fluid diverting block.

In a preferred embodiment, the upstream end of the central fluid diverting block is located behind the downstream end of the side fluid diverting block.

In a specific and preferred embodiment, the flow electrotransfection device further includes an electrode bracket on which the electrode module is placed, wherein the electrode module comprises two planar electrodes which are placed facing each other and spaced apart, the chamber is formed surrounded by the electrode bracket and two of the electrodes, the fluid guiding structure is composed of two side fluid diverting blocks and one central fluid diverting block, wherein the side fluid diverting blocks and the central fluid diverting block are located at an upstream end of the electrodes.

In a more preferred embodiment, the liquid inlet channel is formed in a liquid inlet tube, which is connected to the electrode bracket, and the side fluid diverting block and the central fluid diverting block are connected to inner wall of the liquid inlet tube.

In a more preferred embodiment, the liquid inlet tube is designed with a trumpet-shaped opening, the inner width of which gradually increases along the fluid flowing direction, and the side fluid diverting block and the central fluid diverting block are placed at the trumpet-shaped opening and/or in an area of the chamber adjacent to the trumpet-shaped opening.

In a more preferred embodiment, the liquid outlet channel is formed in a liquid outlet tube, which is connected to the electrode bracket, and the side fluid diverting block and the central fluid diverting block are connected to inner wall of the liquid outlet tube.

In an embodiment, the fluid guiding structure is located in the fluid inlet channel or in an area of the chamber adjacent to the fluid inlet channel. Optionally, the fluid guiding structure is located in the fluid outlet channel or in an area of the chamber adjacent to the fluid outlet channel.

In an embodiment, the fluid guiding structure includes one or more fluid diverting blocks.

In a preferred embodiment, the shape of the cross-section of the fluid diverting block is triangular, trapezoidal, parallelogram-shaped, polygonal, circular, elliptical, wavy or conical.

In an embodiment, the fluid guiding structure includes a diverting blade.

In a preferred embodiment, the fluid guiding structure includes a plurality of diverting blades, one end of each of the diverting blades is connected to each other, and the other end of each of the diverting blades extends outward in a radial shape.

In an embodiment, the electrode module includes a pair of planar electrodes or a needle electrode array.

Compared with the prior art, the invention adopts the above solution and has the following advantages:
There exists a technical problem that flow velocities of fluid at different zones in the flow electrotransfection chamber is uneven, i.e., the flow velocity of fluid in the central zone of the chamber is faster, the flow velocity of fluid in a zone near the chamber wall is slower, which may cause a retention zone to form. Cells in the slow flowing zone and the retention zone could receive higher number of electric shock that may cause cells damage or death. The flow electrotransfection device of present invention solves the problem of uneven flow velocities by adopting a flow electrode structure that can divert fluid flow, improves the accuracy of the number of electric shock that cells would receive, and reduces cell damage and even death from excessive electric shock, thereby improving cell viability and electrotransfection efficiency.

### Brief description of the drawings

In order to illustrate the technical solutions of the present invention more clearly, a brief introduction to the accompanying drawings used in the description of the embodiments is presented below. Obviously, the drawings in the following description are only some examples of embodiments of the present invention, and for those of ordinary skill in the art, other drawings can also be obtained from these drawings without creative effort.
Fig. 1, Schematic diagram of the exploded structure of the flow electrotransfection device of an embodiment of present invention;
Fig. 2(A), Schematic diagram of the designed relative positions among fluid diverting blocks, and the shape of the cross-section of fluid diverting blocks in the fluid guiding structure of an embodiment of present invention;
Fig. 2(B), Schematic diagram of the designed relative positions among fluid diverting blocks and the shape of the cross-section of fluid diverting blocks in the fluid guiding structure of an embodiment of present invention;
Figure 2(C), Schematic diagram of the designed relative positions among fluid diverting blocks and the shape of the cross-section of fluid diverting blocks in the fluid guiding structure of an embodiment of present invention;
Fig. 2 (D), Schematic diagram of the designed relative positions among fluid diverting blocks and the shape of the cross-section of fluid diverting blocks in the fluid guiding structure of an embodiment of the present invention;
Fig. 2 (E), Schematic diagram of the designed relative positions among fluid diverting blocks and the shape of the cross-section of fluid diverting blocks in the fluid guiding structure of an embodiment of the present invention;
Fig. 2 (F), Schematic diagram of the designed relative positions among fluid diverting blocks and the shape of the cross-section of fluid diverting blocks in the fluid guiding structure of an embodiment of the present invention;
Fig. 2 (G), Schematic diagram of the designed relative positions among fluid diverting blocks and the shape of the cross-section of fluid diverting blocks in the fluid guiding structure of an embodiment of the present invention;
Fig. 2 (H), Schematic diagram of the designed relative positions among fluid diverting blocks and the shape of the cross-section of fluid diverting blocks in the fluid guiding structure of an embodiment of the present invention;
Fig. 2 (I), Schematic diagram of the designed relative positions among fluid diverting blocks and the shape of the cross-section of fluid diverting blocks in the fluid guiding structure of an embodiment of the present invention;
Fig. 2 (J), Schematic diagram of the designed relative positions among fluid diverting blocks and the shape of the cross-section of fluid diverting blocks in the fluid guiding structure of an embodiment of the present invention;
Fig. 3, Cross-sectional view of the fluid guiding structure placed at the liquid inlet in the flow electrotransfection device of an embodiment of the present invention;
Fig. 4, Cross-sectional view of the fluid guiding structure placed at a liquid outlet in the flow electrotransfection device of an embodiment of the present invention;
Fig. 5, Cross-sectional view of the fluid guiding structures placed at the liquid inlet and the liquid outlet in the flow electrotransfection device of an embodiment of the present invention;
Fig. 6, Schematic diagram of the exploded structure of the flow electrotransfection device of an embodiment of the present invention;
Fig. 7, Experiment results of electrotransfection of CHO-S cells in buffer containing FITC-Dextran (average molecular weight 500kDa), by using the flow electrotransfection device A of Fig. 2 (F) and the flow electrotransfection device B without a fluid guiding structure, respectively;
Fig. 8, Experiment results of electrotransfection of CHO-S cells with plasmid pcDNA3.1, by using the flow electrotransfection device A of Fig. 2(H) and the flow electrotransfection device B without fluid guiding structure, respectively;
Fig.9, Schematic diagram of flow velocity distribution of fluid in the flow electrotransfection device of an embodiment of the present invention;
Fig. 10, Schematic diagram of the exploded structure of the flow electrotransfection device of an embodiment of the present invention, wherein the fluid guiding structure is not shown;
Fig. 11, Partial enlarged view of Fig. 9;

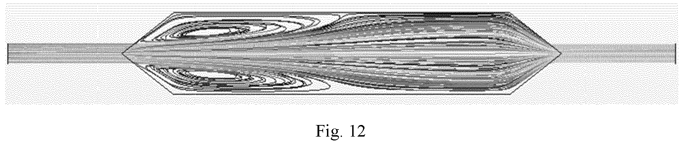

Fig. 12, Schematic diagram of flow velocity distribution of fluid in a flow electrotransfection device in the prior art.

Wherein, 1-electrode bracket, 2-first electrode, 3-second electrode, 4-liquid outlet channel, 40-liquid outlet tube, 5- liquid diverting block, 51-side liquid diverting block, 511-upstream end, 512-downstream end, 52-central liquid diverting block, 521-upstream end, 6-guiding fissure, 7-fluid guiding structure, 8-liquid inlet channel, 80-liquid inlet tube, 81- trumpet-shaped opening, 810-wall, 9-chamber, 10-retention zone, 11-central axis, 12-bracket for diverting blades, 13-diverting blade, 100-fluid.

### Detailed description of the embodiments

The technical solutions in the embodiments of the present invention will be described clearly and completely below. Obviously, the described embodiments are only a part of the embodiments of the present invention, rather than all embodiments. Based on the embodiments of the present invention, all other embodiments obtained by those of ordinary skill in the art without creative efforts shall fall within the protection scope of the present invention.

### Embodiment 1

In the flow electrotransfection device of the present embodiment (Fig. 1), the chamber 9 is formed by the electrode bracket 1, the first electrode 2 and the second electrode 3, and the first electrode 2 and the second electrode 3 are placed in parallel. The upper and lower parts of the electrode bracket 1 are connected to a liquid inlet channel 8 and a liquid outlet channel 4 respectively, and the fluid guiding structure 7 is placed in the area close to the chamber 9 in the liquid inlet channel 8 (Fig. 3).The fluid guiding structure 7 is formed by combining two or more liquid diverting blocks 5 with triangular cross-section, or the liquid diverting blocks 5 with triangular and trapezoidal cross-section, and guiding fissures 6 are formed between the fluid diverting blocks 5. The placement of the fluid diverting blocks 5 is shown in Fig. 2(A) and Fig. 2(B).

When the fluid flows (into the chamber 9) from the liquid inlet channel 8, it encounters the fluid guiding structure 7, a part of the fluid flows to the areas at two sides guided by the two side liquid diverting blocks 5, which drive the fluid in the two side retention zones 10 to flow to the liquid outlet channel 4. A part of the fluid flowing straightly (into the chamber 9) from the liquid inlet channel 8 flows though the guiding fissures 6 in the middle part, making the fast-flowing liquid near the central zone of the chamber 9 to slow down and not to rush directly from the liquid inlet channel 8 to the liquid outlet channel 4. Therefore, the flow velocities of fluid at different zones in the chamber 9 are more even and smooth as a whole, and the electrotransfection of cells in the fluid reaches higher efficiency.

### Embodiment 2

In the flow electrotransfection device of the present embodiment (Fig. 1), the chamber 9 is formed by the electrode bracket 1, the first electrode 2 and the second electrode 3, and the first electrode 2 and the second electrode 3 are placed in parallel. The upper and lower parts of the electrode bracket 1 are connected to a liquid inlet channel 8 and a liquid outlet channel 4 respectively, and the fluid guiding structure 7 is placed in the area close to the chamber 9 in the liquid inlet channel 8 (Fig. 3). The fluid guiding structure 7 is formed by combining two or more liquid diverting blocks 5 with quadrilateral cross-section, or the liquid diverting blocks 5 with quadrilateral and triangular and trapezoidal cross-section, and guiding fissures 6 are formed between the fluid diverting blocks 5. The arrangement of the fluid diverting blocks 5 is shown in Fig. 2(C) and Fig. 2(D).

When the fluid flows (into the chamber 9) from the liquid inlet channel 8, it encounters the fluid guiding structure 7, a part of the fluid flows to the areas at two sides guided by the two side liquid diverting blocks 5, which drive the fluid in the retention zones 10 at two sides to flow to the liquid outlet channel 4, and a part of the fluid flowing straightly (into the chamber 9) from the liquid inlet channel 8 flows though the guiding fissures 6 in the middle part, making the fast-flowing liquid near the central zone of the chamber 9 to slow down and not to rush directly from the liquid inlet channel 8 to the liquid outlet channel 4. Therefore, the flow velocities of fluid at different zones in the chamber 9 are more even and smooth as a whole, and the electrotransfection of cells in the fluid reaches higher efficiency.

### Embodiment 3

In the flow electrotransfection device of the present embodiment (Fig. 1), the chamber 9 is formed by the electrode bracket 1, the first electrode 2 and the second electrode 3, and the first electrode 2 and the second electrode 3 are placed in parallel. The upper and lower parts of the electrode bracket 1 are connected to a liquid inlet channel 8 and a liquid outlet channel 4 respectively, and the fluid guiding structure 7 is placed in the area close to the chamber 9 in the liquid inlet channel 8 (Fig. 3). The fluid guiding structure 7 is formed by combining two or more liquid diverting blocks 5 with circular and triangular cross-section, or the liquid diverting blocks 5 with circular and arc-shaped transitional streamlined cross-section. One or more guiding fissures 6 appear in the center of the arc-shaped liquid diverting block 5 and guiding fissures 6 are formed between the fluid diverting blocks 5. The arrangement of the fluid diverting blocks 5 is shown in Fig. 2(E) and Fig. 2(F).

When the fluid flows (into the chamber 9) from the liquid inlet channel 8, it encounters the fluid guiding structure 7, a part of the fluid flows to the areas at two sides through the two side liquid diverting blocks 5, which drive the fluid in the retention zones 10 at two sides to flow to the liquid outlet channel 4, and a part of the fluid flowing straightly (into the chamber 9) from the liquid inlet channel 8 flows though the guiding fissures 6 in the middle part, making the fast-flowing liquid near the central zone of the chamber 9 to slow down and not to rush directly from the liquid inlet channel 8 to the liquid outlet channel 4. Therefore, the flow velocities of fluid at different zones in the chamber 9 are more even and smooth as a whole, and the electrotransfection of cells in the fluid reaches higher efficiency.

### Embodiment 4

In the flow electrotransfection device of the present embodiment (Fig. 1), the chamber 9 is formed by the electrode bracket 1, the first electrode 2 and the second electrode 3, and the first electrode 2 and the second electrode 3 are placed in parallel. The upper and lower parts of the electrode bracket 1 are connected to a liquid inlet channel 8 and a liquid outlet channel 4 respectively, and the fluid guiding structure 7 is placed in the area close to the chamber 9 in the liquid inlet channel 8 (Fig. 3). The fluid guiding structure 7 is formed by combining two or more liquid diverting blocks 5 with wavy cross-section, and guiding fissures 6 are formed between the fluid diverting blocks 5. The arrangement of the fluid diverting blocks 5 is shown in Fig. 2(G) and Fig. 2(H).

When the fluid flows (into the chamber 9) from the liquid inlet channel 8, it encounters the fluid guiding structure 7, a part of the fluid flows to the areas at two sides through the two side liquid diverting blocks 5, which drive the fluid in the retention zones 10 at two sides to flow to the liquid outlet channel 4, and a part of the fluid flowing straightly (into the chamber 9) from the liquid inlet channel 8 flows though the guiding fissures 6 in the middle part, making the fast-flowing liquid near the central zone of the chamber 9 to slow down and not to rush directly from the liquid inlet channel 8 to the liquid outlet channel 4. Therefore, the flow velocities of fluid at different zones in the chamber 9 are more even and smooth as a whole, and the electrotransfection of cells in the fluid reaches higher efficiency.

### Embodiment 5

In the flow electrotransfection device of the present embodiment (Fig. 1), the chamber 9 is formed by the electrode bracket 1, the first electrode 2 and the second electrode 3, and the first electrode 2 and the second electrode 3 are placed in parallel. The upper and lower parts of the electrode bracket 1 are connected to a liquid inlet channel 8 and a liquid outlet channel 4 respectively, and the fluid guiding structure 7 is placed in the area close to the chamber 9 in the liquid inlet channel 8 (Fig. 3). The fluid guiding structure 7 is formed by combining two or more liquid diverting blocks 5 with irregular polygonal cross-section, and guiding fissures 6 are formed between the fluid diverting blocks 5. The arrangement of the fluid diverting blocks 5 is shown in Fig. 2(I) and Fig. 2(J).

When the fluid flows (into the chamber 9) from the liquid inlet channel 8, it encounters the fluid guiding structure 7, a part of the fluid flows to the areas at two sides through the two side liquid diverting blocks 5, which drive the fluid in the retention zones 10 at two sides to flow to the liquid outlet channel 4, and a part of the fluid flowing straightly (into the chamber 9) from the liquid inlet channel 8 flows though the guiding fissures 6 in the middle part, making the fast-flowing liquid near the central zone of the chamber 9 to slow down and not to rush directly from the liquid inlet channel 8 to the liquid outlet channel 4. Therefore, the flow velocities of fluid at different zones in the chamber 9 are more even and smooth as a whole, and the electrotransfection of cells in the fluid reaches higher efficiency.

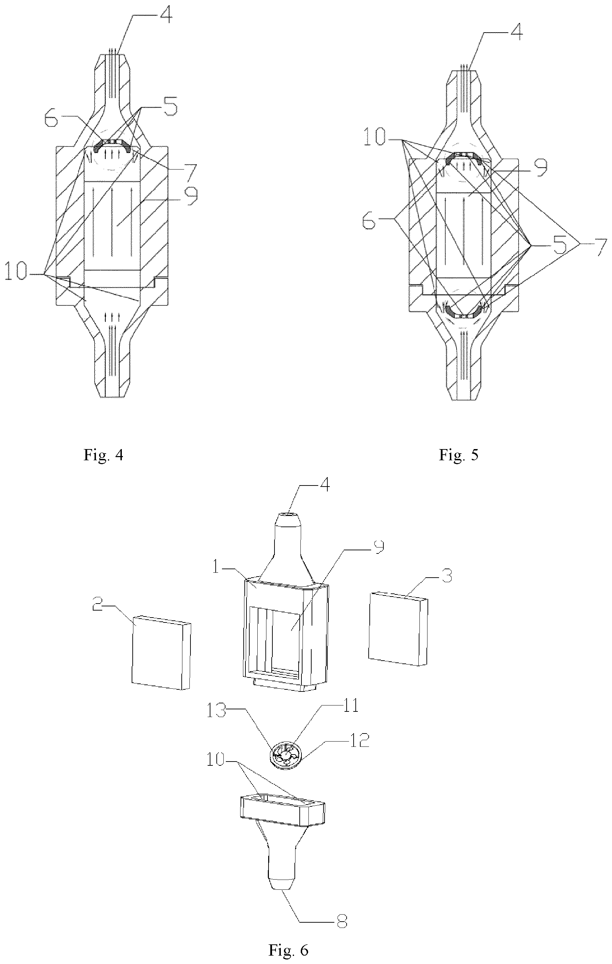

The position of the fluid guiding structure 7 in Embodiment 1-5 can be changed. The fluid guiding structure 7 can be placed in the liquid outlet channel 4, and further in the area close to the chamber 9 in the liquid outlet channel 4. For example, the fluid guiding structure 7 shown in Fig. 2(F) is placed in the liquid outlet channel 4 (Fig. 4).

The position of the fluid guiding structure 7 in Embodiment 1-5 can be changed. The fluid guiding structures 7 can be placed in both the liquid inlet channel 8 and the liquid outlet channel 4, and further in the areas close to the chamber 9 in the liquid inlet channel 8 and the liquid outlet channel 4, respectively. For example, the fluid guiding structures 7 shown in Fig. 2(F) are placed in both the liquid inlet channel 8 and the liquid outlet channel 4 (FIG. 5).

### Embodiment 6

In the flow electrotransfection device of the present embodiment (Fig. 6), the chamber 9 is formed by the electrode bracket 1, the first electrode 2 and the second electrode 3, and the first electrode 2 and the second electrode 3 are placed in parallel. The upper and lower parts of the electrode bracket 1 are connected to a liquid inlet channel 8 and a liquid outlet channel 4 respectively, and the bracket for diverting blades 12 is placed in the area close to the chamber 9 in the liquid inlet channel 8. The central axis 11 and the diverting blades 13 are mounted on the bracket for diverting blades 12.

When the fluid flows (into the chamber 9) from the liquid inlet channel 8, it encounters and flows through the bracket for diverting blades 12, and drives the diverting blades 13 to rotate around the central axis 11, so that the fluid flowing through the diverting blades 13 spreads out in the entire chamber 9, driving the fluid in the retention zones 10 at two sides to flow to the liquid outlet channel 4. The fluid flowing straightly (into the chamber 9) from the liquid inlet channel 8 flows though the diverting blades 13, making the fast-flowing liquid near the central zone of the chamber 9 to slow down, and not to rush directly from the liquid inlet channel 8 to the liquid outlet channel 4. Therefore, the flow velocities of fluid at different zones in the chamber 9 are more even and smooth as a whole, and the electrotransfection of cells in the fluid reaches higher efficiency.

The vertical flow movement is altered to a helical flow movement by using the diverting blades 13, so as to achieve evenness of the flow velocities of fluid in different zones in the chamber 9.

### Embodiment 7

A controlled experiment is set up with the flow electrotransfection device A of the Embodiment 3 and the flow electrotransfection device B without the fluid guiding structure to compare the difference in electrotransfection efficiency. At the same time, a blank controlled group is set up, the treatment of the cells in this group is the same as that of the electrotransfection groups but without electric shock.

CHO-S cells are electrotransfected in buffer containing FITC-Dextran (average molecular weight 500kDa), by using the flow electrotransfection device A shown of Fig. 2(F) in Embodiment 3 and the flow electrotransfection device B without the fluid guiding structure, respectively. Cells in logarithmic growth phase are collected, centrifuged at 300 g for 5 min, and then the supernatant is discarded. Cells are resuspended and washed with DPBS, centrifuged again at 300 g for 5 min, the supernatant is discarded. Cells are then resuspended to a density of 1 × 10⁸/mL with EBEL electrotransfection buffer, and FITC-Dextran is added at 0.5 mg/mL. Immediately after mixing, electrotransfection is performed by using the device A and the device B, respectively (electrotransfection parameters: 220V voltage, 1ms pulse width, interval of 1s, 3 times electric shock). After electrotransfection, cells are inoculated at a viable cell density of 5×10⁶/mL and cultured in shake flasks. After 2 h, histograms of cellular FITC signals for different treatment groups are detected by flow cytometry.

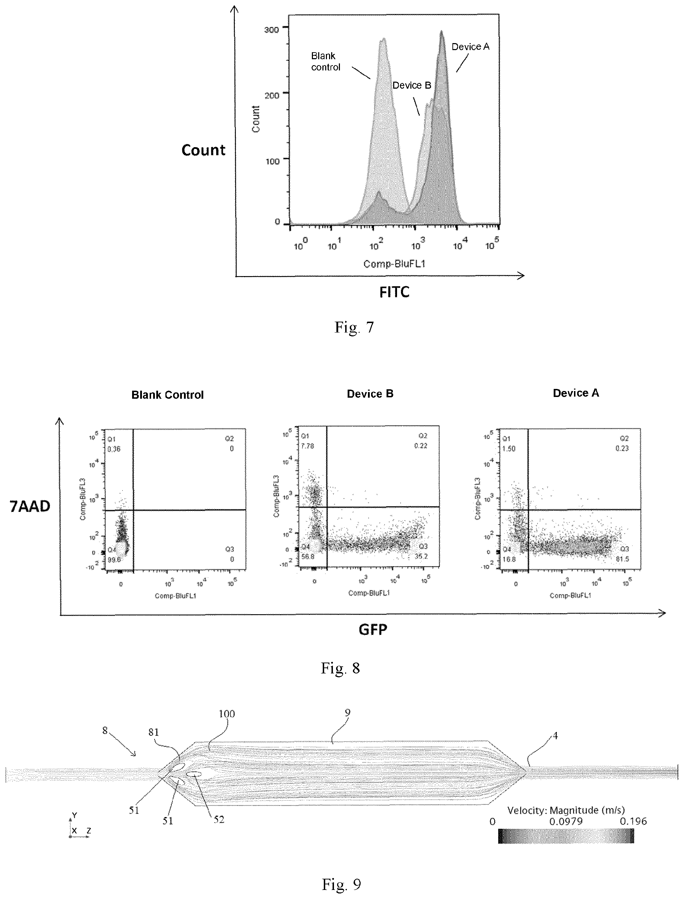

As the results shown by Fig. 7, cells that were electrotransfected using the flow electrotransfection device A shown in Fig. 2(F) of Embodiment 3 of the present invention have a narrower FITC signal peak width, indicating that the number of electric shock the cells had received was more accurate, therefore the transfection efficiency of FITC-Dextran is higher.

### Embodiment 8

A controlled experiment was set up using the flow electrotransfection device A of Embodiment 4 and the flow electrotransfection device B without the fluid guiding structure to compare the differences in cell viability and GFP positive rate after flow electrotransfection. At the same time, a blank controlled group was set up, the treatment of cells was the same as that of the electrotransfection groups but without electric shock.

CHO-S cells were electrotransfected with plasmid pcDNA3.1 using the flow electrotransfection device A shown in Fig. 2(H) in Embodiment 4 and the flow electrotransfection device B without the fluid guiding structure, respectively. Cells in logarithmic growth phase were collected, centrifuged at 300 g for 5 min, the supernatant was discarded. Cells were resuspended and washed with DPBS, centrifuged again at 300 g for 5 min, the supernatant was discarded. Cells were resuspended to a density of 1 × 10⁸/mL with EBEL electrotransfection buffer, and then pcDNA3.1 was added at 100µg/mL. Immediately after mixing, electrotransfection was performed by using the device A and the device B, respectively (electrotransfection parameters: 230V voltage, 600µs pulse width, interval of 1s, 4 times electric shock). After electrotransfection, cells were inoculated at a viable cell density of 4×10⁶/mL and cultured in shake flasks. After 24 h, cells were harvested for 7AAD staining, cell viability was detected by flow cytometry, and GFP positive rates of cells in different groups were detected.

As observed from the results shown in Fig. 8, cells that were electrotransfected using the flow electrotransfection device A shown in Fig. 2(F) in Embodiment 4 of the present invention have higher cell viability and GFP positive rate.

### Embodiment 9

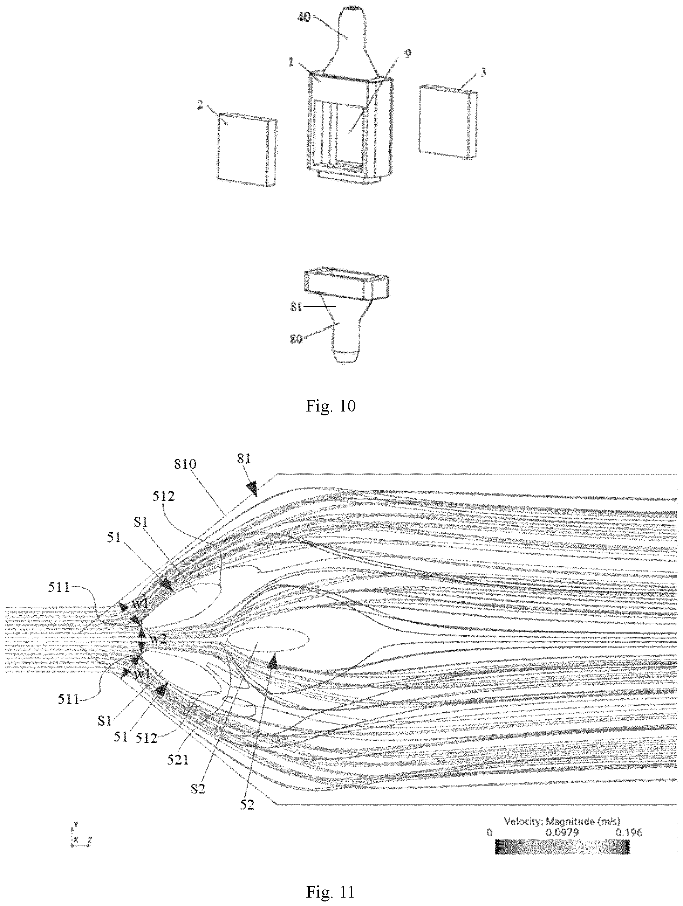

The flow electrotransfection device of the present embodiment is shown in Figs. 9 to 11. The flow electrotransfection device, with the chamber 9 for the fluid 100 to flow though, a liquid inlet channel 8 connected to the chamber 9 and a liquid outlet channel 4 connected to the chamber 9, further includes an electrode bracket 1 and an electrode module for applying an electric field to the fluid 100 in the chamber 9. The electrode module is placed in the electrode bracket 1. The electrode module comprises the planar first electrode 2 and the planar second electrode 3, which are placed in parallel and spaced apart, and the chamber 9 is formed among the electrode bracket 1, the first electrode 2 and the second electrode 3. The liquid inlet channel 8 is formed in a liquid inlet tube 80, which is connected to the electrode bracket 1, and the liquid outlet channel 4 is formed in a liquid outlet tube 40, which is connected to the electrode bracket 1. The liquid inlet tube 80 and the liquid outlet tube 40 are respectively connected to opposite ends of the electrode bracket 1 such as the left-end and the right-end shown in Fig. 9, or the lower end and the upper end shown in Fig. 10.

The flow electrotransfection device further includes the fluid guiding structure 7 for making the flow velocities of the fluid 100 flowing through the central zone and the peripheral zone of the chamber 9 to become more even. The liquid inlet tube 80 is designed with the trumpet-shaped opening 81, the inner width of 810 gradually increases along the direction of fluid flow, and a fluid guiding structure 7 is placed in the trumpet-shaped opening 81. The fluid guiding structure 7 includes two side fluid diverting blocks 51, and a central fluid diverting block 52 located in the middle of the two side fluid diverting blocks 51, all of the fluid diverting blocks are placed in the trumpet-shaped opening 81. Each of the side fluid diverting blocks 51 and the central fluid diverting block 52 has an upstream end 511 that is closer to the liquid inlet channel 8, and a downstream end 512 that is farther away from the liquid inlet channel 8 (Fig. 8 and 11). A first fissure for fluid to flow though is formed between the upstream end 521 of the central fluid diverting block 52 and the upstream end 511 of each of the two side fluid diverting blocks 51 respectively , and a second fissure w1 for fluid to flow though is formed between the upstream end 511 of each of the two side fluid diverting blocks 51 and the wall of the liquid inlet channel 8 (specifically, the inner wall 810 of the trumpet-shaped opening 81 of the liquid inlet tube 80). The side fluid diverting block 51 and the central fluid diverting block 52 are connected to the inner wall of the liquid inlet tube 80, so that they are firmly located in the liquid inlet channel 8.

In the present embodiment, the upstream end 521 of the central fluid diverting block 52 is located at the rear side of the upstream end 511 of the side fluid diverting block 51 and preferably located at the rear side of the downstream end 512 of the side fluid diverting block 51 (Fig. 11). As used herein, the orientation words "front" and "rear" are defined according to the flow direction of fluid as a whole, with the upstream side as "front" and the downstream side as "rear". A third fissure w2, which is roughly equal in width to each of the second fissures w1 at two sides, is formed between the upstream ends 511 of the two side fluid diverting blocks 51. The outer contour of the cross sections S1 of the side fluid diverting block 51 consist of arcs, and the outer contour of the cross section S2 of the central fluid diverting block 52 consists of arcs. Therein, the width of the cross-section S1 of the side fluid diverting block 51 gradually increases and then decreases from its upstream end 511 to its downstream end 512, and the width of the cross-section S2 of the central fluid diverting block 52 gradually increases and then decreases from its upstream end 521 to its downstream end. And the geometrical center of each of the side fluid diverting blocks 51 is equidistant from the geometrical center of the central fluid diverting block 52. In the present embodiment, the center fluid diverting block 52 is flat elliptical, i.e. the rate of change of the width of its cross-section S2 from the upstream end 521 to the middle part is approximately the same as that from the middle part to the downstream end. In some embodiments, the width of the cross-section S2 of the central block 52 changes in the way that the rate of change from the upstream end 521 to the middle part is greater than that from the middle part to the downstream end, meaning that the change in width of the portion from the middle part to the downstream end (i.e. the rear half) of the central fluid diverting block 52 is smaller than that from the upstream end to the middle part (i.e. the front half), so as to further reduce the magnitude of the eddy current in the central zone behind the central fluid diverting block 52.

The flow of fluid through the flow electrotransfection device is simulated and shown in Fig. 9. The fluid in the fluid inlet channel 8 is divided into three tributaries by the upstream ends 511 of the two side fluid diverting blocks 51, and the middle tributary is further divided by the central fluid diverting block 52 at the downstream of the two side fluid diverting blocks 51, and then is guided by each fluid diverting block to form a distribution as shown in Fig. 9. As observed from the simulation results, the fluid 100 can flow through the chamber 9 at a relatively even flow velocity, after being diverted and guided by the three fluid diverting blocks at the trumpet-shaped opening 81. Especially the flow velocity of the fluid 100 in the center of the chamber 9 tends to be similar to that of the fluid 100 at the side zone (near the wall) of the chamber 9, and there is no obvious eddy current, thus resulting in a more even flow velocity of the fluid.

As stated in this specification and the claims, the terms "comprising" and "consist of" only imply the inclusion of clearly identified steps and elements, which does not constitute an exclusive list, and methods or devices may also include other steps or elements. As used herein, the term "and/or" includes any combination of one or more of the associated listed items. It is further understood that the terms "first", "second", etc. are used to describe various information, but the information should not be limited to these terms. These terms are only used to distinguish the same type of information from one another, and do not imply a particular order or level of importance. In fact, the expressions "first", "second" etc. are used completely interchangeably. For example, the first information may also be referred to as the second information, and similarly, the second information may also be referred to as the first information, without departing from the scope of the present disclosure.

The above-mentioned embodiments are only to illustrate the technical concept and characteristics of the present invention, and are preferred embodiments. The purpose of the embodiments is for those who are familiar with the technology to understand the content of the present invention and to implement accordingly, which shall not limit the scope of protection of the present invention.

## Claims

1. A flow electrotransfection device, with a chamber for fluid to flow though, a liquid inlet channel connected to the chamber and a liquid outlet channel connected to the chamber, including an electrode module for applying an electric field to fluid in the chamber, is **characterized in that**, the flow electrotransfection device further includes a fluid guiding structure for evening out flow velocities of fluid flowing through different zones in the chamber.

2. The flow electrotransfection device according to claim 1, is **characterized in that**, the fluid guiding structure, located in the liquid inlet channel, comprises a plurality of side fluid diverting blocks , and a central fluid diverting block located between the side fluid diverting blocks, each of the side fluid diverting blocks and the central fluid diverting block includes an upstream end that is closer to the liquid inlet channel and a downstream end that is further away from the liquid inlet channel, while a first fissure for fluid to flow though is formed between the upstream end of the central fluid diverting block and the upstream end of the side fluid diverting blocks, and a second fissure for fluid to flow though is formed between the upstream end of the side fluid diverting block and the wall of the liquid inlet channel.

3. The flow electrotransfection device according to claim 2, is **characterized in that**, a third fissure, which is roughly equal in width to each of the second fissures, is formed between the upstream ends of the two side fluid diverting blocks.

4. The flow electrotransfection device according to claim 2, is **characterized in that**, the outer contours of the cross-sections of the side fluid diverting block and the central fluid diverting block consist of arcs.

5. The flow electrotransfection device according to claim 4, is **characterized in that**, the width of the cross-section of the side fluid diverting block gradually increases and then decreases from its upstream end to its downstream end, and the width of the cross-section of the central fluid diverting block gradually increases and then decreases from its upstream end to its downstream end.

6. The flow electrotransfection device according to claim 5, is **characterized in that**, the width of the cross-section of the central fluid diverting block changes specifically according to the following rule that the rate of change from the upstream end to the middle part is larger than that from the middle part to the downstream end.

7. The flow electrotransfection device according to claim 5, is **characterized in that**, the distance between the upstream ends of the side fluid diverting blocks is smaller than that between the downstream ends of the side fluid diverting blocks.

8. The flow electrotransfection device according to claim 2, is **characterized in that**, the geometric center of each of the side fluid diverting blocks is equidistant from the geometric center of the central fluid diverting block.

9. The flow electrotransfection device according to claim 2, is **characterized in that**, the liquid inlet channel and the liquid outlet channel are located at the front and rear sides of the chamber respectively, and the upstream end of the central fluid diverting block is located behind the upstream end of the side fluid diverting block.

10. The flow electrotransfection device according to claim 9, is **characterized in that**, the upstream end of the central fluid diverting block is located behind the downstream end of the side fluid diverting block.

11. The flow electrotransfection device according to any one of claims 2-10, is **characterized in that**, the flow electrotransfection device further includes an electrode bracket on which the electrode module is placed, wherein the electrode module comprises two planar electrodes that are placed facing each other and spaced apart, the chamber is formed surrounded by the electrode bracket and two of the electrodes, the fluid guiding structure is composed of two side fluid diverting blocks and one central fluid diverting block, wherein the side fluid diverting blocks and the central fluid diverting block are located at an upstream end of the electrodes.

12. The flow electrotransfection device according to claim 11, is **characterized in that**, the liquid inlet channel is formed in a liquid inlet tube, which is connected to the electrode bracket, and the side fluid diverting block and the central fluid diverting block are connected to inner wall of the liquid inlet tube.

13. The flow electrotransfection device according to claim 12, is **characterized in that**, the liquid inlet tube is designed with a trumpet-shaped opening, the inner width of which gradually increases along the fluid flowing direction, and the side fluid diverting block and the central fluid diverting block are placed at the trumpet-shaped opening and/or in an area of the chamber adjacent to the trumpet-shaped opening.

14. The flow electrotransfection device according to claim 12, is **characterized in that**, the liquid outlet channel is formed in a liquid outlet tube, which is connected to the electrode bracket, and the side fluid diverting blocks and the central fluid diverting block are connected to inner wall of the liquid outlet tube.

15. The flow electrotransfection device according to claim 1, is **characterized in that**, the fluid guiding structure is located in the fluid inlet channel or in an area of the chamber adjacent to the fluid inlet channel, and/or, the fluid guiding structure is located in the fluid outlet channel or in an area of the chamber adjacent to the fluid outlet channel.

16. The flow electrotransfection device according to claim 1, is **characterized in that**, the fluid guiding structure includes one or more fluid diverting blocks.

17. The flow electrotransfection device according to claim 16, is **characterized in that**, the shape of the cross-section of the fluid diverting block is triangular, trapezoidal, parallelogram-shaped, polygonal, circular, elliptical, wavy or conical.

18. The flow electrotransfection device according to claim 1, is **characterized in that**, the fluid guiding structure includes a diverting blade.

19. The flow electrotransfection device according to claim 18, is **characterized in that**, the fluid guiding structure includes a plurality of diverting blades, one end of each of the diverting blades is connected to each other, and the other end of each of the diverting blades extends outward in a radial shape.

20. The flow electrotransfection device according to claim 1, is **characterized in that**, the electrode module includes a needle electrode array or a pair of planar electrodes.
